# FASCICULE DE BREVET EUROPEEN

(11) **EP 1 523 562 B1**
(45) Date de publication et mention de la délivrance du brevet: **12.10.2005**
(21) Numéro de dépôt: 03750832.2
(22) Date de dépôt: 22.07.2003
(51) Int. Cl.: C12N 15/86, C07K 14/705

(54) **EXPRESSION DE RECEPTEURS A 7 DOMAINES TRANSMEMBRANAIRES EN SYSTEME BACULOVIRUS/CELLULES D'INSECTES**
EXPRESSION VON REZEPTOREN MIT 7 TRANSMEMBRANDOMÄNEN IN EINEM BACULOVIRUS/INSEKTENZELLEN SYSTEM
EXPRESSION RECEPTORS WITH 7 TRANSMEMBRANE DOMAINS IN BACULOVIRUS- INSECT CELL SYSTEM

(30) Priorité: 24.07.2002 FR 0209377
(43) Date de publication de la demande: 20.04.2005
(73) Titulaire: CENTRE NATIONAL DE LA RECHERCHE SCIENTIFIQUE (CNRS), 75016 Paris (FR); Université de Montpellier II, 34095 Montpellier Cedex 5 (FR)
(72) Inventeur: DEVAUCHELLE, Gérard, F-30380 SAINT-CHRISTOL-LEZ-ALES (FR); DEMAILLE, Jacques, F-34980 MONTFERRIER-SUR-LEZ (FR); FERRAZ, Conception, F-34090 MONTPELLIER (FR); MATARAZZO, Valéry, F-73000 BASSENS (FR); RONIN, Catherine, F-13960 SAUSSET-LES-PINS (FR); CERUTTI, Martine, F-30380 SAINT-CHRISTOL-LEZ-ALES (FR)
(74) Mandataire: Vialle-Presles, Marie José
(86) Numéro de dépôt international: PCT/FR2003/002309
(87) Numéro de publication internationale: WO 2004/011658

(56) Documents cités:
- GAT U ET AL: "OLFACTORY RECEPTOR PROTEINS EXPRESSION, CHARACTERIZATION AND PARTIAL PURIFICATION" EUROPEAN JOURNAL OF BIOCHEMISTRY, BERLIN, DE, vol. 225, no. 3, 1994, pages 1157-1168, XP002926155 ISSN: 0014-2956
- RAMING K ET AL: "CLONING AND EXPRESSION OF ODORANT RECEPTORS" NATURE, MACMILLAN JOURNALS LTD. LONDON, GB, vol. 361, 28 janvier 1993 (1993-01-28), pages 353-356, XP002029937 ISSN: 0028-0836 cité dans la demande
- BREER H ET AL: "Expression and functional analysis of olfactory receptors." ANNALS OF THE NEW YORK ACADEMY OF SCIENCES, vol. 855, 30 novembre 1998 (1998-11-30), pages 175-181, XP009006842 International Symposium;San Diego, California, USA; July 7-12, 1997, Nov. 30, 1998 New York Academy of Sciences 2 East 63rd Street, New York, New York 10021, USA ISBN: 1-57331-139-1 cité dans la demande
- GRUENEWALD SYLVIA ET AL: "Glycosylation, palmitoylation, and localization of the human D-2S receptor in baculovirus-infected insect cells." BIOCHEMISTRY, vol. 35, no. 48, 1996, pages 15149-15161, XP002234106 ISSN: 0006-2960
- GRUENEWALD SYLVIA ET AL: "In vivo reconstitution of dopamine D-2S receptor-mediated G protein activation in baculovirus-infected insect cells: Preferred coupling to G-i1 versus G-i2." BIOCHEMISTRY, vol. 35, no. 48, 1996, pages 15162-15173, XP002234107 ISSN: 0006-2960
- MIN M K ET AL: "Transcriptional analyses of baculovirus polyhedrin and foreign gene expression relative to baculovirus p10 mRNA levels." THE JOURNAL OF GENERAL VIROLOGY. ENGLAND OCT 1991, vol. 72 ( Pt 10), octobre 1991 (1991-10), pages 2551-2556, XP009006988 ISSN: 0022-1317
- GRISSHAMMER R ET AL: "Overexpression of integral membrane proteins for structural studies." QUARTERLY REVIEWS OF BIOPHYSICS. ENGLAND AUG 1995, vol. 28, no. 3, août 1995 (1995-08), pages 315-422, XP009006949 ISSN: 0033-5835
- MASSOTTE DOMINIQUE: "G protein-coupled receptor overexpression with the baculovirus-insect cell system: a tool for structural and functional studies." BIOCHIMICA ET BIOPHYSICA ACTA. NETHERLANDS 17 FEB 2003, vol. 1610, no. 1, 17 février 2003 (2003-02-17), pages 77-89, XP001146155 ISSN: 0006-3002

### SEQUENCE LISTING

<110> CNRS
   UNIVERSITE DE MONTPELLIER II
   DEVAUCHELLE, Gérard
   DEMAILLE, Jacques
   FERRAZ, Conception
   MATARAZZO, Valéry
   RONIN, Catherine
   CERRUTI, Martine
<120> EXPRESSION DE RECEPTEURS A 7 DOMAINES TRANSMEMBRANAIRES EN SYSTEME BACULOVIRUS/CELLULES D'INSECTES
<130> MJPbv644/68
<150> FR 0209377
   <151> 2002-07-24
<160> 13
<170> PatentIn version 3.1
<210> 1
   <211> 109
   <212> DNA
   <213> Artificial sequence
<220>
   <223> séquence codant pour la fusion EGT-épitope FLAG
<400> 1
   atgactattc tctgctggct tgcactgctg tctacgctta ctgctgttaa cgcggactac 60
   aaggacgatg atgacaaagc catggctgct cggtaccctg cacgagctc 109
<210> 2
   <211> 34
   <212> DNA
   <213> Artificial sequence
<220>
   <223> amorce PCR
<400> 2
   taagaagctt gccaccatgg aggggaaaaa tctg 34
<210> 3
   <211> 39
   <212> DNA
   <213> Artificial sequence
<220>
   <223> amorce PCR
<400> 3
   taacggtacc gcggccgcct aaggggaatg aattttccg 39
<210> 4
   <211> 39
   <212> DNA
   <213> Artificial sequence
<220>
   <223> amorce PCR
<400> 4
   caataagctt ccatggctat gtatttgtgt ctcagcaac 39
<210> 5
   <211> 39
   <212> DNA
   <213> Artificial sequence
<220>
   <223> amorce PCR
<400> 5
   taacggtacc gcggccgctt aagccactga tttagagtg 39
<210> 6
   <211> 60
   <212> DNA
   <213> Artificial sequence
<220>
   <223> amorce PCR
<400> 6
   ttacgatatc agatctgcca ccatgtaccc ctacgacgtc cctgactacg ccatggcccg 60
<210> 7
   <211> 34
   <212> DNA
   <213> Artificial sequence
<220>
   <223> amorce PCR
<400> 7
   ctataagctt tcacagcagg ttgatctcgt ccag 34
<210> 8
   <211> 70
   <212> DNA
   <213> Artificial sequence
<220>
   <223> amorce PCR
<400> 8
   ttacgatatc agatctgcca ccatgtaccc ctacgacgtc cctgactacg ccatggggtg 60
   tttgggcaac 70
<210> 9
   <211> 34
   <212> DNA
   <213> Artificial sequence
220>
   <223> amorce PCR
<400> 9
   ctataagctt tcacaagagt tcgtactgct tgag 34
<210> 10
   <211> 10
   <212> PRT
   <213> Artificial sequence
<220>
   <223> motif conservé au début du domaine transmembranaire II des récepteurs olfactifs
<400> 10
   Pro Met Tyr Leu Phe Leu Gly Asn Leu Ser
   1 5 10
<210> 11
   <211> 10
   <212> PRT
   <213> Artificial sequence
<220>
   <223> motif conservé à la fin du domaine transmembranaire IV et au début de la boucle intracellulaire i2 des récepteurs olfactifs
<400> 11
   Met Ala Tyr Asp Arg Tyr Val Ala Ile Cys
   1 5 10
<210> 12
   <211> 6
   <212> PRT
   <213> Artificial sequence
<220>
   <223> motif conservé au début du domaine transmembranaire VI des récepteurs olfactifs
<400> 12
   Phe Ser Cys Ser Ser His
   1 5
<210> 13
   <211> 6
   <212> PRT
   <213> Artificial sequence
<220>
   <223> motif conservé dans le domaine transmembranaire VII des récepteur s olfactifs
<400> 13
   Pro Met Leu Asn Pro Phe
   1 5

## Description

L'invention est relative à la production, dans un système baculovirus/cellules d'insecte, de récepteurs à sept domaines transmembranaires, et notamment de récepteurs couplés aux protéines G.

La super-famille des récepteurs à sept domaines transmembranaires couplés aux protéines G (RCPGs), comprend notamment de nombreux récepteurs membranaires de neurotransmetteurs, de neuropeptides et d'hormones. Ils ont tous la même organisation structurale, à savoir une seule chaîne polypeptidique comprenant sept domaines hydrophobes qui traversent la double couche lipidique membranaire.

Parmi les récepteurs couplés aux protéines G, les récepteurs olfactifs (ORs) représentent une très large famille et constituent chez les vertébrés la famille numériquement la plus importante (ZOZULYA et al., Genome Biology, 6(2), 2001 : http://www.genomebiology.com/2001/2/6/research/0018.1; ZHANG et FIRESTEIN, Nat. Neurosci., 5(2), 124-133, 2002). Ils sont principalement localisés à la surface des membranes des cellules neuroréceptrices (neurones olfactifs) de l'épithélium olfactif, mais leur expression dans d'autres tissus a également été rapportée. Un très grand nombre de gènes codant pour des récepteurs olfactifs ont été identifiés, permettant d'évaluer à plusieurs centaines, voire plusieurs milliers, le nombre de types différents de récepteurs olfactifs chez un mammifère. Depuis l'isolement initial d'ADNc codant pour des ORs à partir de l'épithélium olfactif de rat (BUCK et AXEL, Cell, 65 (1), 175-187, 1991), des gènes codant pour des ORs putatifs ont été clonés à partir de nombreuses espèces de vertébrés, dont l'homme, et d'invertébrés, (pour revue, voir MOMBAERTS, Annual Review of Neuroscience, 22, 487-509, 1999). Les séquences totales ou partielles de ces gènes sont accessibles sur les bases de données, et sont notamment regroupées dans la base ORDB accessible à l'adresse http://ycmi.med.yale.edu/senselab/ordb/(SKOUFOS *et al.*, Nucl. Acids Res., 28, 341-343, 2000).

Les critères, établis d'après les données de séquences disponibles, qui sont habituellement utilisés pour classer un récepteur à sept domaines transmembranaires parmi les récepteurs olfactifs sont les suivants :
- la taille de la molécule : il s'agit de récepteurs de petite taille (environ 300-400 acides aminés), dans lesquels les boucles intra- et extracellulaires et le segment terminal sont très courts (environ 15 à 40 acides aminés pour les boucles intra- et extracellulaire, et environ 20 à 30 acides aminés pour le segments N-terminal) ;
- l'homologie des séquences en acides aminés : certains motifs d'acides aminés sont très conservés entre les différents récepteurs olfactifs (BUCK et AXEL, Cell, 65 (1), 175-187, 1991 ; PILPEL et LANCET, Protein Science, 8 (5), 969-977, 1999). Il s'agit notamment des motifs : PMYLFLGNLS (SEQ ID NO : 10) au début du domaine transmembranaire II, MAYDRYVAIC (SEQ ID NO : 11) à la fin du domaine transmembranaire IV et au début de la boucle intracellulaire i2, SY à la fin du domaine transmembranaire V, FSCSSH (SEQ ID NO : 12) au début du domaine transmembranaire VI et PMLNPF (SEQ ID NO : 13) dans le domaine transmembranaire VII. Généralement, la coexistence de ces motifs est suffisante pour classer une séquence de vertébré à sept domaines transmembranaires dans la famille des ORs. D'une manière plus générale, la plus grande conservation entre les différents récepteurs olfactifs se situe au niveau des domaines transmembranaires II, VI, VII et de la boucle intracellulaire i2. Au contraire, les domaines transmembranaires III, IV et V sont des régions hypervariables.

La liaison d'une molécule odorante à un récepteur olfactif entraîne l'activation de protéines G qui stimulent la cascade enzymatique conduisant à la production d'AMP cyclique (AMPc). L'AMPc induit à son tour l'ouverture de canaux ioniques sodiques et calciques, entraînant la dépolarisation de la membrane du neurone olfactif, et induisant ainsi un influx nerveux qui transmet le signal au bulbe olfactif.

Un second mécanisme de transduction olfactif a également été proposé, faisant intervenir comme second messager l'inositol triphosphate (IP₃) au lieu de l'AMPc (BOEKHOFF *et al*., EMBO Journal, 9 (8), 2453-2458, 1990 ; SCHILD *et al*., Journal of Neurophysiology, 73 (2), 862-866, 1995). Des expérimentations récentes indiquent toutefois que, physiologiquement, la transduction olfactive serait principalement générée par la voie de l'AMPc et non par celle d'IP₃ (BELLUSCIO *et al*., Neuron., 20 (1), 69-81, 1998 ; WONG *et al*., Neuron., 27 (3), 487-497, 2000 ; BRUNET *et al*., Neuron., 17 (4), 681-693, 1996).

Les protéines G qui ont été identifiées comme impliquées dans la transduction du signal olfactif par la voie de l'AMPc sont Gαs et Gαolf. Dans le cas de la transduction par la voie IP₃, il a été rapporté que les protéines Go ou Gq (FADOOL *et al*., Chemical Senses, 20, 489-498, 1995 ; SCHANDAR *et al*., J. Biol. Chem., 273, 16669-16677, 1998) pourraient être impliquées.

L'expression en système hétérologue constitue potentiellement un outil majeur pour la caractérisation fonctionnelle et la bioingénierie des récepteurs couplés aux protéines G.

Des récepteurs couplés aux protéines G ont été exprimés sous forme fonctionnelle dans différents systèmes hétérologues : on citera notamment le système baculovirus/cellules d'insectes, qui a été utilisé par exemple pour exprimer des récepteurs adrénergiques, des récepteurs muscariniques, des récepteurs à la sérotonine, aux canabinoïdes, à l'ocytocine, à la substance P, etc. (PARKER *et al*., J. Biol. Chem., 266 (1), 519-527, 1991 ; RICHARDSON et HOSEY, J. Biol. Chem., 267 (31), 11149-22255, 1992 ; VASUDEVAN *et al*., FEBS Lett., 311 (1), 7-11, 1992 ; BUTKERAIT *et al*., J. Biol. Chem., 270 (31), 18691-18699, 1995 ; NOWELL *et al*., Biochemical Pharmacology, 55 (11), 1893-1905, 1998 ; GIMPL *et al*., Biochemistry, 34 (42), 13794-13801, 1995 ; NISHIMURA *et al*., Journal of Receptor & Signal Transduction Research, 18 (1), 51-65, 1998), ainsi que pour reconstituer le couplage RCPG/protéine G, par coexpression sous contrôle du promoteur de la polyédrine, d'un récepteur et d'une protéine G (BUTKERAIT *et al*., J. Biol. Chem., 270 (31), 18691-18699, 1995 ; BARR *et al*., J. Biol. Chem., 272 (4), 2223-2229-1997).

L'expression en système hétérologue est d'un intérêt tout particulier dans le cas des récepteurs olfactifs, qui sont en majorité des récepteurs orphelins, dont les ligands ne sont pas connus.

Quelques récepteurs olfactifs ont ainsi été exprimés dans des cellules de mammifères ou d'insectes. Dans le cas des cellules d'insecte, RAMING *et al.* (Nature, 361 (3410), 353-356, 1993) ont exprimé le récepteur OR5 de rat dans des cellules Sf9 après infection par un baculovirus recombinant, et ont observé des augmentations transitoires du second messager IP₃ dans les cellules infectées en réponse à une stimulation par le lyral ou le lilial. Des résultats similaires ont été rapportés par BREER *et al*. (Annals of the New York Academy of Sciences, 855, 175-181, 1998) et GAT et al. (European Journal of Biochemistry, 225(3), 1157-1168, 1994).

Cependant, une limitation au développement à l'utilisation de systèmes d'expression hétérologues, en particulier dans le cas des récepteurs olfactifs, résulte d'un niveau d'expression relativement faible de récepteurs fonctionnels à la surface cellulaire. Il est supposé que ce problème résulte d'un mauvais adressage à la membrane plasmique quand ces récepteurs sont exprimés dans des cellules autres que les neurones olfactifs matures. Il semble que des interactions intra-moléculaires au niveau de la 3^{ème} boucle intracellulaire pourraient être à l'origine de la rétention de ces protéines dans les compartiments intracellulaires (GIMELBRANT *et al.*, Journal of Neurochemistry, 72 (6), 2301-2311, 1999).

Pour améliorer l'adressage à la membrane des récepteurs olfactifs dans des cellules de mammifères, il a été proposé de les fusionner avec un peptide signal hétérologue. Des banques d'ORs chimériques, fusionnés avec le peptide signal d'un récepteur de la rhodopsine ou de la sérotonine, ont été exprimés dans des cellules HEK293 (KRAUTWURST *et al*., Cell, 95 (7), 917-926, 1998 ; WETZEL *et* *al*., Journal of Neuroscience, 19 (17), 7426-7433, 1999). Il a ainsi été observé que l'exposition de ces cellules transfectées à des mélanges odorants conduisait à des augmentations transitoires du calcium intracellulaire, et que cette réponse était amplifiée quand les ORs étaient co-exprimés avec la protéine G_{α15-16} (également dénommée G_{α16}), un sous-type de G_{q} universel (KRAUTWURST *et al*., précité).

Il a également été proposé d'exprimer des récepteurs olfactifs dans des neurones olfactifs matures, afin de permettre d'une part leur adressage et leur insertion correcte dans la membrane plasmique, et d'autre part de fournir un système de seconds messagers adaptés, capables de générer un signal suffisamment important pour être détecté (Brevet US 5 993 778).

Les Inventeurs ont maintenant mis au point un système améliorant l'expression de récepteurs couplés aux protéines G, et notamment de récepteurs olfactifs, dans des cellules d'insectes.

Ils ont émis l'hypothèse que l'adressage à la membrane de ces récepteurs pouvait être plus efficace si on les exprimait sous contrôle d'un promoteur plus faible que le promoteur polyédrine habituellement utilisé pour l'expression de gènes hétérologues dans un baculovirus.

Ils ont testé différents promoteurs, et ont constaté que l'utilisation d'un promoteur de la polyédrine partiellement tronqué afin de diminuer son activité, permettait d'améliorer l'adressage des récepteurs à la membrane plasmique des cellules hôte d'insecte, et d'obtenir ainsi l'expression en surface de récepteurs fonctionnels.

La présente invention a pour objet une cassette d'expression comprenant :
a) un promoteur dérivé du promoteur de la polyédrine d'un baculovirus par délétion de tout ou partie de la région dudit promoteur s'étendant des positions -1 à -12 par rapport au site d'initiation de la traduction de la polyédrine ;
b) une séquence codant pour un récepteur à sept domaines transmembranaires, placée sous contrôle transcriptionnel dudit promoteur.

Avantageusement, pour obtenir le niveau d'expression optimal dans le cadre de la mise en oeuvre de la présente invention, on utilise un promoteur dans lequel la portion délétée comprend au moins la région s'étendant des positions -1 à -5 par rapport au site d'initiation de la traduction de la polyédrine.

Selon un mode de réalisation préféré de la présente invention, ladite cassette d'expression comprend en outre, en amont de la séquence b), une séquence c) codant pour un peptide signal.

A titre d'exemples non limitatifs de séquences codant pour des peptides signal utilisables pour la mise en oeuvre de la présente Invention, on citera le peptide signal de l'Ecdystéroide UDP Glucosyl Transférase (EGT) du baculovirus AcNPV, le peptide signal de la lactotransferrine bovine, etc.

Ladite cassette d'expression peut également comprendre en amont ou en aval de la séquence b) codant pour le récepteur à sept domaines transmembranaires, une séquence d) codant pour un peptide étiquette facilitant la détection du récepteur recombinant exprimé. A titre d'exemples non limitatifs de peptides étiquette utilisables pour la mise en oeuvre de la présente Invention, on citera l'épitope FLAG, l'épitope HA, etc.

Une cassette d'expression conforme à l'invention peut être construite pour exprimer n'importe quel récepteur à sept domaines transmembranaires. De manière particulièrement avantageuse, ledit récepteur est un récepteur olfactif.

La présente invention a également pour objet un procédé pour exprimer un récepteur à sept domaines transmembranaires dans une cellule d'insecte, caractérisé en ce que l'on infecte ladite cellule d'insecte avec un baculovirus recombinant comprenant une cassette d'expression telle que définie ci-dessus. Avantageusement, ladite cassette d'expression est insérée en remplacement du promoteur et du gène natifs de la polyédrine dudit baculovirus.

Selon un mode de mise en oeuvre préféré de la présente invention, on exprime également dans la même cellule d'insecte, une protéine G qui peut être marquée à son extrémité N-terminale par un peptide étiquette, par exemple l'épitope HA, sans que cela gêne l'expression de la protéine à la surface de la cellule d'insecte ou son couplage au récepteur.

Selon une disposition particulièrement avantageuse de ce mode de mise en oeuvre, ladite protéine G est exprimée sous contrôle du promoteur du gène P10 d'un baculovirus.

Les Inventeurs ont en effet constaté que l'utilisation du promoteur du gène P10, dont l'expression est un peu plus précoce que celle du gène de la polyédrine, permet d'exprimer un récepteur à sept domaines transmembranaires dans une cellule contenant déjà une quantité importante de protéine G, et ainsi d'optimiser le couplage entre ledit récepteur et ladite protéine.

La co-expression du récepteur à sept domaines transmembranaires et de ladite protéine G peut être effectuée en co-infectant une cellule d'insecte par deux baculovirus recombinants : l'un exprimant le récepteur dans une cassette d'expression conforme à l'invention, et l'autre exprimant ladite protéine G sous contrôle du promoteur de la protéine P10.

Toutefois, de manière particulièrement avantageuse, on utilisera un baculovirus double-recombinant, comprenant :
- une cassette d'expression conforme à l'invention, et
- une séquence codant pour une protéine G placée sous contrôle transcriptionnel du promoteur du gène P10 qui peut également comprendre en amont, une séquence codant pour un peptide étiquette, par exemple l'épitope HA.

La présente Invention a également pour objet des vecteurs recombinants, portant au moins une cassette d'expression conforme à l'invention telle que définie ci-dessus.

Dans ce cadre, la présente Invention englobe en particulier :
- des plasmides de transfert, portant un insert comprenant : une cassette d'expression conforme à l'invention, telle que définie ci-dessus, et de part et d'autre de cette cassette, des séquences de baculovirus homologues de celles des régions flanquant le gène de la polyédrine chez le baculovirus dans lequel on souhaite insérer la cassette ;
- des baculovirus recombinants contenant une cassette d'expression conforme à l'invention ; de manière préférée, il s'agit de baculovirus double-recombinants, comprenant en outre une séquence codant pour une protéine G, sous contrôle transcriptionnel du promoteur du gène P10. Ces baculovirus peuvent notamment être obtenus, de manière classique, par recombinaison homologue entre un plasmide de transfert conforme à l'invention et le génome d'un baculovirus. Dans le cas des baculovirus double-recombinants, on effectue une étape supplémentaire de recombinaison homologue entre un plasmide de transfert comprenant une séquence codant pour une protéine G, flanquée de séquences de baculovirus homologues de celles flanquant la séquence codant pour la protéine P10 chez le baculovirus concerné.

Pour la mise en oeuvre de la présente invention, on peut utiliser les outils classiques de clonage et d'expression de gènes d'intérêt dans un système baculovirus/cellules d'insecte, tels que ceux décrits par exemple dans BACULOVIRUS EXPRESSION VECTORS : A LABORATORY MANUAL [O'REILLY *et al*., Freeman and Cie, New York, (1994)], ainsi que dans un grand nombre de Brevets ou Demandes de Brevets, tels que, par exemple, la Demande EP 0 651 815, la Demande EP 0 638 647 ou la Demande PCT WO 95/20672.

La présente invention a également pour objet des cellules d'insectes infectées par un baculovirus recombinant conforme à l'invention ; ces cellules expriment à leur surface des récepteurs à sept domaines transmembranaires fonctionnels, capables de se coupler soit à des protéines G endogènes de baculovirus, dans le cas où ils sont exprimés seuls, soit aux protéines G exogènes co-exprimées dans la même cellule.

Les cellules d'insectes selon la présente invention peuvent être utilisées pour l'étude et la caractérisation des récepteurs à sept domaines transmembranaires, notamment pour vérifier la fonctionnalité de récepteurs putatifs, identifier les ligands de récepteurs orphelins, ou étudier des mécanismes impliqués dans l'activation du récepteur, la transduction du signal, par exemple l'identification de la ou des protéine(s) G partenaire(s) du récepteur concerné, l'étude du couplage à ces protéines, et de la fonction du complexe ainsi formé, etc..

Le signal résultant de la liaison de ces récepteurs avec leur(s) ligand (s) peut être détecté par différentes techniques qui sont connues en elles-mêmes. Par exemple, on peut détecter des variations du calcium intracellulaire, notamment par imagerie calcique, ou des variations des seconds messagers, inositol phosphate ou AMP cyclique ; on peut également détecter la phosphorylation et/ou l'activité kinase des protéines impliquées dans la cascade de transduction, etc..

La présente invention a ainsi notamment pour objet :
* Un procédé pour identifier un récepteur à sept domaines transmembranaires fonctionnel, et/ou identifier la protéine G partenaire d'un récepteur à sept domaines transmembranaires, caractérisé en ce qu'il comprend les étapes suivantes :
   - la mise en contact d'une cellule d'insecte conforme à l'invention exprimant un récepteur ou une combinaison récepteur/protéine G à tester avec un mélange de ligands potentiels dudit récepteur ;
   - la détection du signal résultant de la liaison dudit récepteur avec l'un des ligands présents dans ledit mélange.
* Un procédé pour identifier un ligand d'un récepteur à sept domaines transmembranaires, caractérisé en ce qu'il comprend les étapes suivantes :
   - la mise en contact de chaque ligand à tester avec une cellule d'insecte conforme à l'invention, exprimant un récepteur à sept domaines transmembranaires fonctionnel, et une protéine G partenaire dudit récepteur ;
   - l'identification du ou des ligands recherchés, par détection du signal résultant de leur liaison avec ledit récepteur.
* Un procédé pour identifier un récepteur à sept domaines transmembranaires répondant à un ligand d'intérêt, caractérisé en ce qu'il comprend les étapes suivantes :
   - la mise en contact dudit ligand avec une cellule d'insecte conforme à l'invention exprimant un récepteur à sept domaines transmembranaires à tester et une protéine G partenaire dudit récepteur ;
   - l'identification du ou des récepteurs recherchés, par détection du signal résultant de leur liaison avec ledit ligand d'intérêt.

Dans ce cadre, on peut notamment utiliser des cellules d'insecte conformes à l'invention pour exprimer des récepteurs à sept domaines transmembranaires mutants, et cribler le ou les mutants reconnaissant un ligand d'intérêt.

De manière particulièrement avantageuse, les cellules d'insectes selon la présente invention sont utilisées comme décrit ci-dessus, pour l'étude et la caractérisation de récepteurs olfactifs.

Les ligands des récepteurs olfactifs peuvent être des molécules odorantes, des protéines de transport des odorants (OBP pour : « olfactory binding proteins »), des complexes molécule odorante/OBP, ou des molécules synthétiques telles que les cyclodextrines utilisées en parfumerie (savons, bougies, papiers, etc) comme fixateurs d'odorants relâchant très progressivement l'odorant volatile.

Ainsi, on peut utiliser des cellules d'insecte conformes à l'invention pour étudier la fonctionnalité de récepteurs olfactifs putatifs, et/ou de différentes combinaisons récepteur/protéine G, notamment en testant leur réponse à des mélanges de molécules odorantes, des mélanges d'OBPs, des mélanges de complexes molécule odorante/OBP, ou des mélanges de molécules synthétiques telles que définies ci-dessus.

Lorsqu'un récepteur fonctionnel a été identifié, on peut utiliser des cellules d'insecte conformes à l'invention exprimant ledit récepteur et une protéine G partenaire dudit récepteur pour identifier le ou les ligands du récepteur concerné, en testant séparément la réponse de ce récepteur à différentes molécules odorantes ou complexes molécule odorante/OBP. On peut également utiliser des cellules d'insectes conformes à l'invention exprimant des récepteurs olfactifs fonctionnels et des protéines G partenaires desdits récepteurs pour identifier ceux qui répondent à un ligand d'intérêt, en testant séparément la réponse de chacun de ces récepteurs à différentes molécules odorantes ou complexes molécule odorante/OBP.

Des cellules d'insecte conformes à l'invention exprimant un récepteur olfactif fonctionnel dont le ligand a été identifié peuvent avantageusement être utilisées pour l'obtention de biocapteurs, permettant la détection de molécules odorantes, notamment pour l'analyse et le contrôle qualité de composants volatils, au nombre desquels figurent des produits aromatiques d'intérêt, et/ou pour la détection de produits risquant d'affecter les qualités organoleptiques, ou pour la détection de produits potentiellement nocifs.

Par exemple, la présente invention peut être mise en oeuvre pour :
- exprimer et purifier des récepteurs olfactifs orphelins chez différentes espèces (par exemple homme, animaux domestiques, etc.);
- produire des anticorps spécifiques de ces récepteurs utilisables dans le typage immunocytochimique de neuroépithélium prélevé en clinique, par exemple dans le cas d'une greffe de cellules engainantes dans la moelle épinière lésée;
- cribler des molécules odorantes ou des OBPs (naturelles ou recombinantes) pour une utilisation pharmacologique;
- cribler des récepteurs olfactifs mutants fonctionnels ou des OBPs mutées à l'aide de molécules odorantes d'intérêt, par exemple des arômes alimentaires, des fumets, des parfums, etc.

La présente Invention sera mieux comprise à l'aide du complément de description qui va suivre, qui se réfère à des exemples non-limitatifs d'expression, conformément à l'invention, de récepteurs olfactifs fonctionnels dans des cellules d'insecte.

### EXEMPLE 1 : OBTENTION DE BACULOVIRUS RECOMBINANTS EXPRIMANT DES RECEPTEURS OLFACTIFS HUMAINS SOUS CONTROLE D'UN PROMOTEUR DE LA POLYEDRINE MODIFIE.

### Construction des vecteurs de transfert.

### Vecteur pGmAc 217 :

Ce vecteur contient un insert, obtenu à partir du fragment EcoRI I de 7 kb environ du baculovirus AcMNPV, comprenant la région de la polyédrine, par délétion du fragment -8 à +502 (par rapport au site d'initiation de la traduction de la polyédrine) et remplacement de la portion délétée par un lieur BglII. L'insert ainsi obtenu comprend donc un promoteur *polh* de la polyédrine délété de 8 pb (positions -1 à -8).

### Insertion d'un peptide signal et d'une séquence de marquage :

Un oligonucléotide est inséré dans le vecteur pGmAc 217, immédiatement en aval du promoteur *polh* modifié. La séquence de cet oligonucléotide est la suivante :

Cet oligonucléotide comprend une séquence de 54 pb (soulignée) codant pour le peptide signal de l'Ecdystéroide UDP Glucosyl Transférase (EGT) du baculovirus AcNPV (GenBank M22619), suivie par la séquence codant pour l'épitope FLAG (en italiques), elle-même suivie de deux sites de restriction uniques NcoI et KpnI (en gras).

### Gènes codant pour les récepteurs olfactifs

Les gènes sans introns codant pour les récepteurs olfactifs putatifs OR-209 et OR-210 à partir du chromosome humain 17 (p13.3) sont amplifiés par PCR en utilisant le cosmide No. ICRF105cF06137 (GenBank N°HSU53583).

Les amorces oligonucléotidiques sens (S) et antisens (AS) suivantes sont utilisées :

Les amplifications sont effectuées dans le mélange réactionnel suivant :

10 ng de la préparation d'ADN du cosmide, 10 mM de Tris-HCl (pH 8,3), 50 mM de KCl, 2,5 mM de MgCl₂, 0,001% de gélatine, 0,2 mM de dNTP, 1,5 U de mélange de polymérases EXPAND™ (ROCHE MOLECULAR BIOCHEMICALS) et 100 pmol de chacune des amorces ;
et selon le programme suivant : 94°C (90 sec, 1 cycle), 94°C (20 sec), 50°C (25 sec) et 72°C (90 sec) (40 cycles) ; 72°C (120 sec, 1 cycle).

Les deux produits de PCR sont digérés avec NcoI/KpnI et les fragments résultant sont sous-clonés entre les sites NcoI et KpnI du vecteur pGmAc 217 contenant le peptide signal.

Les vecteurs de transfert obtenus, dénommés pGmAc 217-209 et pGmAc 217-210 contiennent respectivement la séquence codant pour le récepteur OR-209 et la séquence codant pour le récepteur OR-210.

### Construction des baculovirus recombinants

Les virus recombinants sont obtenus par co-transfection de cellules Sf9 avec l'ADN du baculovirus sauvage AcMNPV, et l'ADN du vecteur de transfert pGmAc 217-209 ou pGmAc 217-210.

Les cellules Sf9 de *Spodoptera frugiperda* (ATCCCRL 1711) sont cultivées en flacon plastique de 25 ou 75 cm² et maintenues à 28°C dans un milieu TC100 (GIBCO-BRL) contenant 5% de sérum bovin foetal (GIBCO-BRL). Des sous-cultures sont effectuées deux fois par semaine.

L'ADN de baculovirus de type sauvage AcMNPV est préparé à partir de particules virales.

La co-transfection est effectuée par lipofection, en incubant 2 x 10⁶ cellules avec 3 ml de milieu sans sérum contenant 500 ng d'ADN de baculovirus et 10 µg de vecteur de transfert, mélangés avec 40 µl de DOTAP (ROCHE, France). Après 4 heures d'incubation à 28°C, le mélange de transfection est éliminé et remplacé par 5 ml de milieu supplémenté de sérum. Les baculovirus recombinants sont récupérés à partir du surnageant des cellules transfectées après 5 jours d'incubation à 28°C.

Les baculovirus recombinants sont isolés par trois étapes de purification par plage de lyse et sélectionnés sur la base d'un phénotype ob- (absence de corps d'inclusion), puis multipliés par des infections cellulaires successives. Les baculovirus recombinants dénommés ci-après AcMNPV209 et AcMNPV210 contiennent respectivement la séquence codant pour le récepteur OR-209 et la séquence codant pour le récepteur OR-210.

Les baculovirus recombinants sont stockés à 4°C jusqu'à leur utilisation.

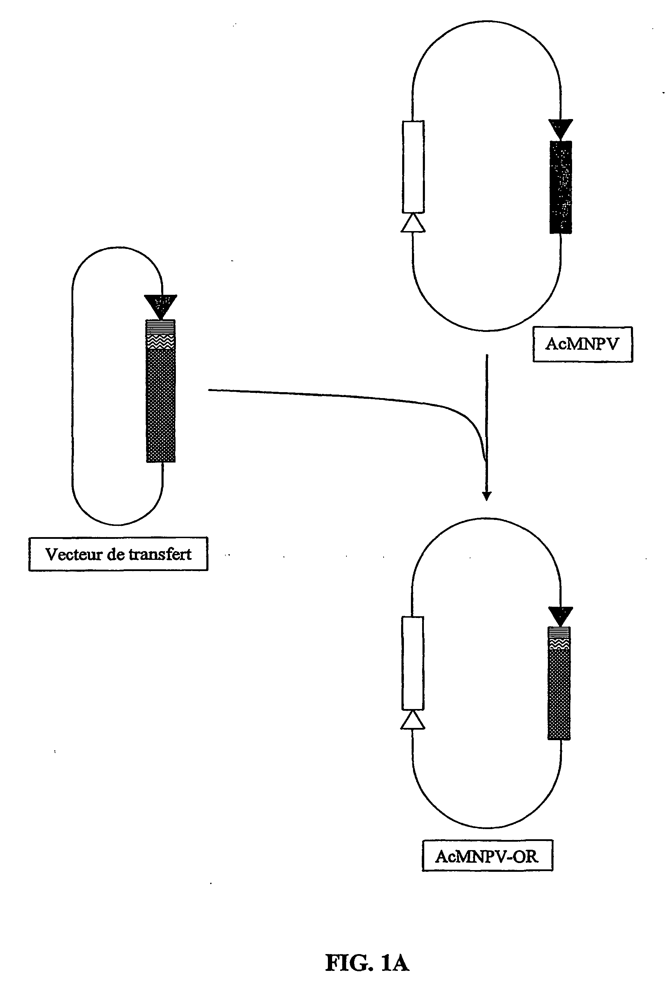

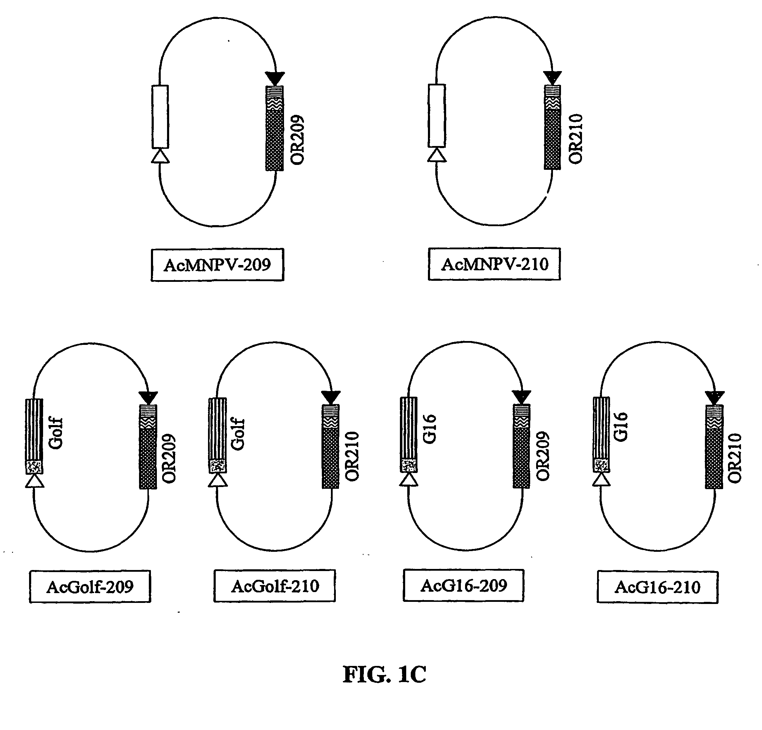

La Figure 1 A représente un schéma général des différentes étapes de construction d'un baculovirus recombinant comprenant une séquence codant pour un récepteur OR, associé au peptide signal du gène EGT et à la séquence de marquage de l'épitope FLAG. Les baculovirus AcMNPV209 et AcMNPv210 sont représentés sur la Figure 1 C.

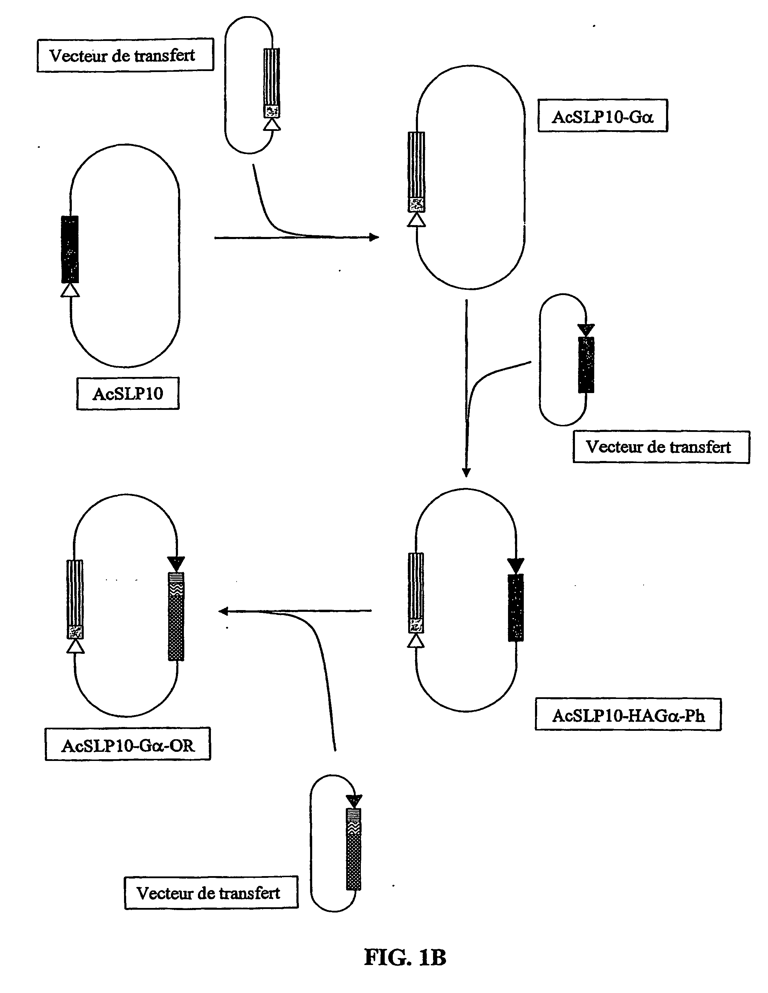

### Légende de la Figure 1 :

■ Polyédrine
▼ Promoteur polyédrine
□ P10
Δ Promoteur P10
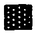 Récepteur olfactif
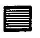 Peptide signal EGT
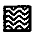 Epitope FLAG
□ Epitope HA
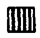 Protéine G

### EXEMPLE 2 : OBTENTION D'UN BACULOVIRUS DOUBLE-RECOMBINANT EXPRIMANT UN RECEPTEUR OLFACTIF HUMAIN SOUS CONTROLE D'UN PROMOTEUR DE LA POLYEDRINE MODIFIE, ET UNE PROTEINE G SOUS CONTROLE DU PROMOTEUR P10.

### Construction des vecteurs de transfert.

### Vecteur hôte :

On utilise le vecteur p119 (LEMEULLE et al., FEBS Lett., 423, 159-166, 1998) contenant les sites de clonage unique BglII et HindIII localisés en aval du promoteur p10 du baculovirus.

### Gènes codant pour des protéines G

### Protéine G_{α16}

L'ADNc codant pour la protéine G_{α16} a été obtenu à partir d'une banque d'ADNc de la lignée promyélocytique de la leucémie humaine HL60 (CLONTECH, France). La séquence de marquage par l'épitope HA est introduite par PCR.

Les oligonucléotides sens (S) et anti-sens (AS) qui ont été utilisés sont les suivants :

L'amplification est effectuée dans le mélange réactionnel suivant :

10 ng de préparation d'ADNc, 10 mM de Tris-HCl (pH 8,3), 50 mM de KCl, 2,5 mM de MgCl₂, 0,001% de gélatine, 0,2 mM de dNTP, 1,5 U de mélange de polymérases EXPAND™ (ROCHE MOLECULAR BIOCHEMICALS) et 100 pmol de chacune des amorces ;
et selon le programme suivant : 94°C (60 sec, 1 cycle), 94°C (45 sec), 58°C (45 sec) et 72°C (90 sec) (35 cycles) ; 72°C (120 sec, 1 cycle).

### Protéine G_{αolf}

L'ADNc codant pour la protéine G_{αolf} a été cloné par RT-PCR à partir d'une préparation d'ARN total de cerveau entier fournie par le Dr. Gilles TOUMANIANTZ (IPMC, Nice, France). Les ARNm sont obtenus en incubant (5 mn à 70°C et 5 mn à 4°C) 4 µg d'ARN total avec 1 µg d'amorce oligonucléotidique dT (INVITROGEN) dans un volume final de 15 µl. 10 µl de la préparation d'ARNm sont alors utilisés comme échantillon pour la transcription inverse en ADNc en utilisant le kit « cDNA cycle » d'INVITROGEN (Pays Bas). La rétro-transcription est réalisée à 42°C pendant 70 mn et la réaction est inactivée à 94°C pendant 5 mn. L'ADNc synthétisé est utilisé comme matrice pour les amplifications PCR ultérieures.

La séquence de marquage par l'épitope HA est introduite par PCR.

Les oligonucléotides sens (S) et anti-sens (AS) utilisés pour cette PCR sont les suivants :

Les conditions d'amplification PCR sont identiques à celles utilisées pour G_{α16}.

Les deux produits de PCR sont digérés avec BglII/HindIII et les fragments résultant sont sous-clonés entre les sites BglII et HindIII du vecteur p119.

Les vecteurs de transfert obtenus, dénommés p119-HA-G_{α16} et p119-HA-G_{olf} pGmAc 217-209 contiennent respectivement la séquence codant pour HA-G_{α16}, et la séquence codant pour HA-G_{olf}.

### Construction des baculovirus recombinants

### Baculovirus recombinants exprimant la protéine G_{α16} ou la protéine G_{αolf} sous contrôle du promoteur du gène P10.

Des cellules Sf9 de *Spodoptera frugiperda* cultivées comme décrit à l'exemple 1 ci-dessus, sont co-transfectées avec l'ADN du baculovirus AcSLP10, et l'un des vecteurs p119-HA-G₁₆ ou p119-HA-G_{olf}.

Le baculovirus AcSLP10 (CHAABIHI *et tal*., J. Virol., 67, 2664-2671, 1993) est un baculovirus modifié qui possède un seul promoteur tardif fort (P10), qui contrôle l'expression de la séquence codant la polyédrine.

La co-transfection est effectuée par lipofection, comme décrit à l'exemple 1 ci-dessus. Les baculovirus recombinants obtenus, dénommés AcSLP10G₁₆ et AcSLP10G_{olf} expriment respectivement p119-HA-G₁₆ ou p119-HA-G_{olf}, sous contrôle du promoteur p10. Ces baculovirus sont multipliés comme décrit à l'exemple 1 ci-dessus.

### Baculovirus double-recombinants.

Les cellules Sf9 de *Spodoptera frugiperda* sont co-transfectées avec l'ADN du baculovirus AcSLP10, et l'un des vecteurs p119-HA-G₁₆ ou p119-HA-G_{olf}, et le vecteur de transfert pGmAcI50 (DEVAUCHELLE et CERUTTI, Les Baculovirus d'Insectes : Vecteurs d'expression de gènes étrangers, Regard sur la Biochimie, n°5, C2, 1991) qui contient la séquence codant pour la polyédrine sous contrôle de son propre promoteur. Les baculovirus recombinants exprimant les protéines G et la polyédrine, sont sélectionnés sur la base de leur phénotype ob+ (présence de corps d'inclusion), et multipliés. Les baculovirus recombinants obtenus, exprimant respectivement p119-HA-G₁₆ ou p119-HA-G_{olf}, sous contrôle du promoteur p10, sont dénommés AcSLP10G₁₆Ph et AcSLP10G_{olf}Ph.

L'ADN de chacun des baculovirus AcSLP10G₁₆Ph ou AcSLP10G_{olf}Ph est utilisé pour co-transfecter des cellules Sf9 avec l'ADN du baculovirus pGmAc 217-209 ou du baculovirus pGmAc 217-210. Les baculovirus double-recombinants obtenus sont sélectionnés sur la base d'un phénotype viral ob-. Ces baculovirus double-recombinants sont dénommés comme AcG₁₆-209 ; AcG_{olf}-210 ; AcG₁₆-209 ; AcG_{olf}-210.

La Figure 1 B représente un schéma général des différentes étapes de construction d'un baculovirus double recombinant comprenant une séquence codant pour une protéine G associée à la séquence de marquage de l'épitope HA sous contrôle du promoteur P10, et une séquence codant pour un récepteur OR, associé au peptide signal du gène EGT et à la séquence de marquage de l'épitope FLAG, sous contrôle du promoteur modifié du gène polyédrine. Les baculovirus AcG₁₆-209 , AcG_{olf}-210, AcG₁₆-209 et AcG_{olf}-210 sont représentés sur la Figure 1 C.

### EXEMPLE 3 : EXPRESSION DE RECEPTEURS OLFACTIFS ET DE PROTEINES G DANS LES CELLULES D'INSECTE.

Les cellules Sf9 sont récoltées 36-48 heures après infection. La production des récepteurs olfactifs ou de protéines G recombinantes est évaluée par transfert immunoélectrophorétique, en utilisant les anticorps dirigés contre leur étiquette épitopique FLAG ou HA.

Les cellules infectées par les baculovirus AcMNPV209 et AcMNPV210 expriment des protéines reconnues par l'anticorps anti-FLAG, et de poids moléculaire correspondant à celui des ORs.

Les cellules infectées par les baculovirus AcSLP10G₁₆ et AcSLP10G_{olf} expriment des protéines reconnues par l'anticorps anti-HA, et de poids moléculaire correspondant à celui des protéines G.

Les cellules infectées par les baculovirus double-recombinants AcG₁₆-209 , AcG_{olf}-210, AcG₁₆-209 et AcG_{olf}-210 expriment les 2 types de protéines.

La localisation cellulaire des ORs et des protéines G a été étudiée par immunofluorescence 36 heures après l'infection. Les ORs sont détectés par un anticorps primaire anti-FLAG et un anticorps secondaire marqué au FITC. Les protéines G sont détectées par un anticorps anti-HA marqué à la rhodamine.

Les cellules marquées sont observées en microscopie confocale. Dans les cellules infectées par les baculovirus AcMNPV209 et AcMNPV210, on observe une localisation du marquage à la surface cellulaire, et une répartition ponctuelle typique des récepteurs membranaires. Dans les cellules infectées par les baculovirus AcSLP10G₁₆ et AcSLP10G_{olf} on observe une localisation cytoplasmique sous-membranaire du marquage. Dans les cellules infectées par les baculovirus double-recombinants AcG₁₆-209 , AcG_{olf}-210, AcG₁₆-209 et AcG_{olf}-210, on observe une co-localisation du récepteur avec chaque protéine G co-exprimée. Aucune différence majeure entre OR17-209 et OR17-210 n'est observée en ce qui concerne une liaison préférentielle avec G_{olf} ou G₁₆. Il apparaît donc que ces deux récepteurs sont capables de se coupler aux deux protéines G dans les cellules d'insecte.

### EXEMPLE 3 : FONCTIONNALITE DES RECEPTEURS EXPRIMES DANS LES CELLULES D'INSECTE.

Dans une première série d'expérimentations, la possibilité de mesurer la réponse calcium dans les cellules Sf9 en utilisant la technique de l'imagerie calcique a été testée.

Le protocole utilisé pour les essais est le suivant :

Les cellules Sf9 sont cultivées sur des plaques de 24 puits contenant des lamelles couvre-objet de 12 mm. Avant les expérimentations, le milieu de culture est éliminé et lavé par un tampon contenant 10 mM de NaCl, 60 mM de KCl, 25 mM de MgCl₂, 1,8 mM de CaCl₂, 4 mM de D-glucose, 110 mM de sucrose et 10 mM d'acide 2-(N-morpholino) éthanesulfonique ; le pH est ajusté à 6,2 à température ambiante avec la base TRIZMA.

Les cellules sont incubées pendant 1 heure à température ambiante dans le noir avec une solution de MBS contenant du fura 2-AM (4 µM) (sonde moléculaire). Les cellules sont ensuite lavées avec un excès de MBS sans fura 2-AM et montées dans une chambre COVERWELL (POLYLABO, France) (diamètre : 9 mm ; épaisseur : 2,5 mm ; section : 22,5).

La chambre est placée sous le microscope, et les cellules sont perfusées sous gravité avec 0,85 ml/mn (3,78 cm/mn) à température ambiante (23°C).

Les mesures de fluorescence fura-2 sont réalisées en utilisant un système d'illumination de longueur d'onde multi-voies POLYCHROME II T.I.L.L PHOTONICS GmbH, (PLANEGG) pour l'extinction. Le microscope (OLYMPUS) est équipé avec un objectif de distance longue portée (X20). Les distributions spatio-temporelles de Ca²⁺ sont étudiées en utilisant une caméra interligne PCO. La longueur d'onde d'excitation du colorant varie alternativement entre 340 et 380 nm et est adaptée au temps d'exposition de la caméra (20-40 msec ; binning : 2). L'acquisition et le calcul des images de fluorescence sont réalisés en utilisant le logiciel T.I.L.L-VISION. Tous les signaux sont rapportés au bruit de fond. Les rapports de fluorescence (f₃₄₀/f₃₈₀) sont calculés pour les variations de Ca²⁺ intracellulaires.

L'effet des produits suivants sur la variation du Ca²⁺ intracellulaire a été testé :
- l'ionophore 4-bromo-A23187, utilisé à une concentration de 5 µM ;
- l'octopamine, utilisée à une concentration de 50 µM, comme contrôle de la capacité des cellules Sf9 à augmenter la concentration en calcium intracellulaire en réponse à l'activation du récepteur à l'octopamine qui est un récepteur couplé aux protéines G/IP3 naturellement présent dans les cellules Sf9 ;
- le mélange HENKEL 100 (HENKEL), qui est un mélange de 100 composés d'arôme différents à la même concentration (1% p/p). Cette solution est diluée au 1/10000 dans le milieu de culture avant utilisation.

Tous les produits sont ajoutés dans la perfusion au moyen d'un distributeur manuel.

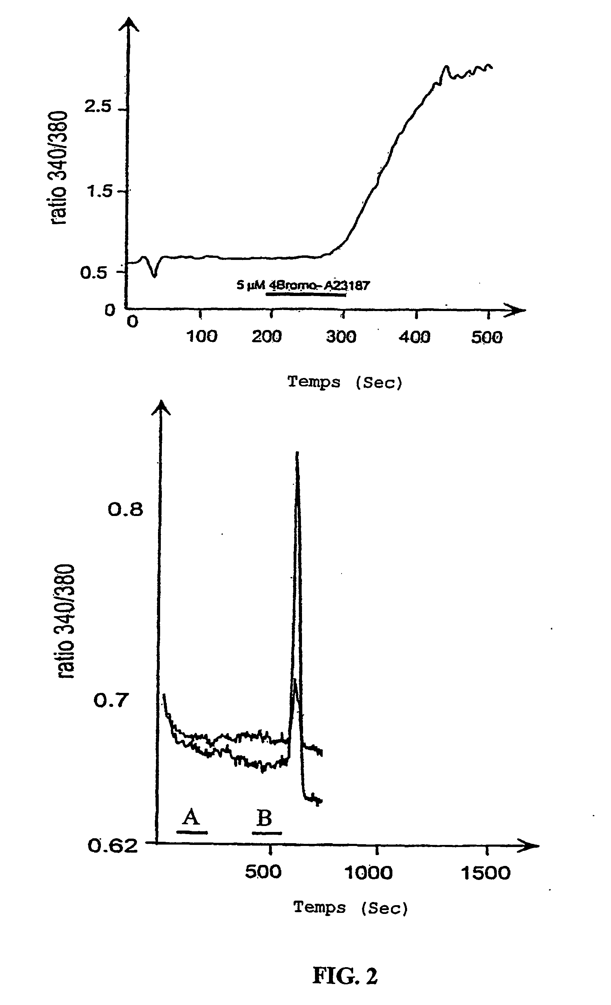

Les résultats obtenus sur les cellules Sf9 non-infectées sont illustrés par la Figure 2. Les signaux Ca²⁺ résultent de la moyenne effectuée sur toutes les cellules dans le champ de la caméra (signal gris) et de la moyenne pour les cellules répondantes (signal noir) (n=4). On observe que l'ionophore 4-bromo A23187 induit une forte augmentation du calcium intracellulaire (graphique du haut). En revanche le mélange odorant HENKEL 100 (graphique du bas, A) ne produit pas de changement du calcium intracellulaire, alors que l'octopamine (graphique du bas, B) induit une augmentation du calcium intracellulaire. Ces résultats montrent que les cellules Sf9 ne possèdent pas de récepteurs olfactifs endogènes dont l'activité pourrait interférer avec celle de récepteurs recombinants exprimés dans ces cellules.

Dans une seconde série d'expérimentation, la capacité des récepteurs OR17-209 et OR17-210 humains, seuls ou co-exprimés avec les protéines G, à répondre aux molécules odorantes a été testée.

Les cellules Sf9 sont infectées avec les baculovirus AcMNPV, AcMNPV209, AcMNPV210, AcG₁₆-209, AcG₁₆-210, AcG_{olf}-209, ou AcG_{olf}-210, et 36 heures après l'infection les réponses cellulaires au mélange HENKEL 100 (10 µM) ou à l'octopamine (50 µM) sont enregistrées par imagerie calcique, comme décrit ci-dessus pour les cellules non-infectées.

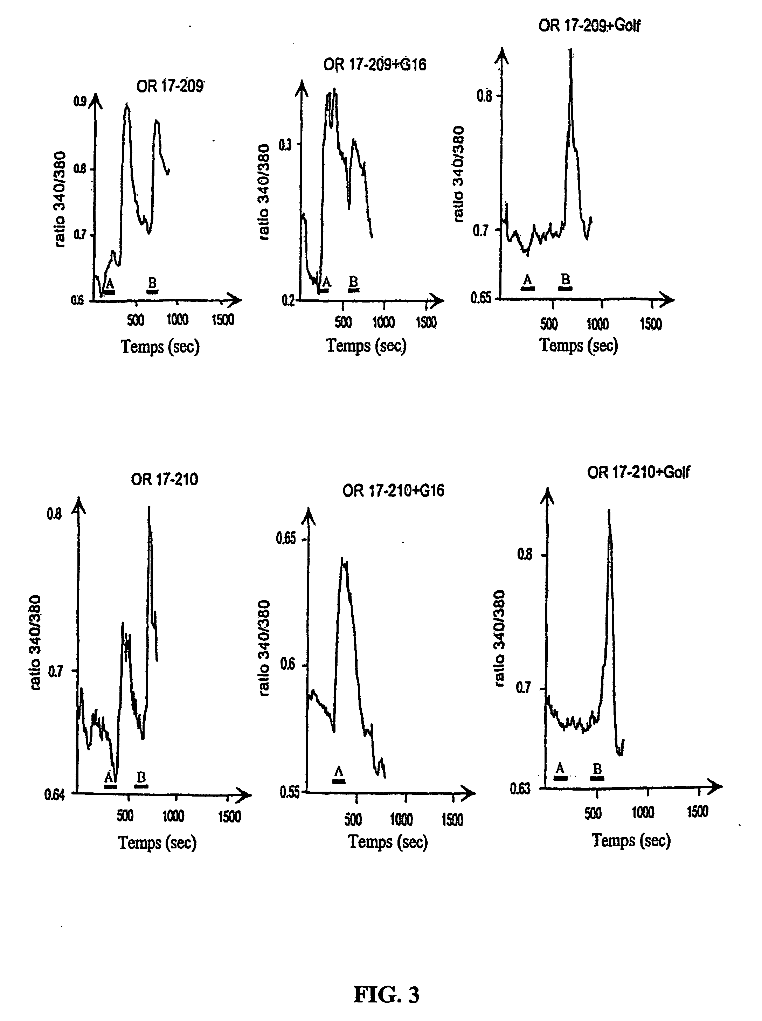

Les résultats sont illustrés par la Figure 3. Les signaux Ca²⁺ résultent de la moyenne à partir des cellules répondantes à l'intérieur du champ de la caméra [récepteur OR17-209 (n=12)/OR17-210 (n=6)/OR17-210-G₁₆ (n=10)/OR 17-209-G₁₆ (n=15)/OR17-209-G_{olf} (n=6)/OR17-210-G_{olf} (n=4)].
A : stimulation par le mélange HENKEL 100 ;
B : stimulation par l'octopamine.

Ces résultats montrent que le mélange odorant HENKEL 100 induit un signal [Ca²⁺]ᵢ transitoire pour les deux récepteurs OR17-209 et OR17-210 exprimés seuls ou co-exprimés avec la protéine G₁₆. En revanche, on n'observe aucune augmentation du calcium intracellulaire dans le cas des récepteurs exprimés avec la protéine G_{olf}. Dans toutes ces cellules l'octopamine induit une augmentation du calcium intracellulaire.

Alors que la co-expression des ORs dans les cellules Sf9 avec la protéine G_{α16} conserve la réponse calcium, leur co-expression avec les protéines G_{olf} entraîne une disparition de cette réponse. Ceci démontre que dans les cellules d'insecte, la réponse aux molécules odorantes est basée sur la libération de Ca²⁺ due au couplage des récepteurs olfactifs aux protéines G endogènes.

Les résultats ci-dessus montrent clairement que les récepteurs olfactifs OR17-209 et OR17-210 humains peuvent être fonctionnellement exprimés dans des cellules Sf9, seuls ou avec la protéine G_{α16}.

Quand les récepteurs olfactifs sont exprimés avec la protéine G_{αolf} aucun changement de [Ca²⁺]ᵢ n'est observé à la suite de l'application de molécules odorantes. Cela pourrait être expliqué par le fait que l'expression de G_{αolf} dans les cellules Sf9 détourne la cascade de signalisation vers une voie qui devient indétectable par l'imagerie calcique. Dans les neurones olfactifs, il est connu que la liaison des molécules odorantes aux récepteurs olfactifs provoque via la protéine G_{olf}, la production d'AMPc qui ouvre directement des canaux calciques contrôlés par les nucléotides cycliques (CNG) dans la membrane plasmique. Ce type de canal est probablement absent des cellules d'insecte Sf9, ce qui mène à l'impossibilité de détecter une réponse calcium quand les récepteurs olfactifs sont co-exprimés avec la protéine G_{αolf} dans ces cellules Sf9. Ces résultats montrent également que les récepteurs olfactifs co-exprimés avec la protéine G_{αolf} se couplent de préférence à celle-ci plutôt qu'aux protéines G endogènes.

## Revendications

1. Cassette d'expression comprenant :
a) un promoteur dérivé du promoteur de la polyédrine d'un baculovirus par délétion de tout ou partie de la région dudit promoteur s'étendant des positions -1 à -12 par rapport au site d'initiation de la traduction de la polyédrine ;
b) une séquence codant pour un récepteur à sept domaines transmembranaires, placée sous contrôle transcriptionnel dudit promoteur.

2. Cassette d'expression selon la revendication 1, **caractérisée en ce qu'**elle comprend en outre, en amont de la séquence b) une séquence codant pour un peptide signal.

3. Cassette d'expression selon une quelconque des revendications 1 ou 2, **caractérisée en ce que** ledit récepteur à sept domaines transmembranaires est un récepteur olfactif.

4. Procédé pour exprimer un récepteur à sept domaines transmembranaires dans une cellule d'insecte, **caractérisé en ce que** l'on infecte ladite cellule d'insecte avec un baculovirus recombinant comprenant une cassette d'expression selon une quelconque des revendications 1 à 3.

5. Procédé selon la revendication 4, **caractérisé en ce que** l'on exprime également dans la même cellule d'insecte, une protéine G.

6. Procédé selon la revendication 5, **caractérisé en ce que** ladite protéine G est exprimée sous contrôle du promoteur du gène P10 d'un baculovirus.

7. Procédé selon la revendication 6, **caractérisé en ce que** l'on utilise un baculovirus double-recombinant, comprenant :
- une cassette d'expression selon une quelconque des revendications 1 à 3 ; et
- une séquence codant pour une protéine G placée sous contrôle transcriptionnel du promoteur du gène P10 dudit baculovirus.

8. Baculovirus recombinant comprenant une cassette d'expression selon une quelconque des revendications 1 à 3.

9. Baculovirus recombinant selon la revendication 8, **caractérisé en ce que** ladite cassette d'expression est insérée en remplacement du promoteur et du gène de la polyédrine dudit baculovirus.

10. Baculovirus recombinant selon la revendication 9, **caractérisé en ce que** ledit baculovirus comprend en outre une séquence codant pour une protéine G placée sous contrôle transcriptionnel du promoteur du gène P10.

11. Cellule d'insecte infectée par un baculovirus recombinant selon une quelconque des revendications 8 à 10.

12. Utilisation d'une cellule d'insecte selon la revendication 11, pour déterminer la fonctionnalité d'un récepteur à sept domaines transmembranaires putatif.

13. Utilisation d'une cellule d'insecte selon la revendication 11 pour identifier le(s) ligand(s) d'un récepteur à sept domaines transmembranaires orphelin.

14. Utilisation d'une cellule d'insecte selon la revendication 11 pour identifier un (des) récepteur(s) à sept domaines transmembranaires capables de se lier à un ligand d'intérêt.

15. Utilisation selon une quelconque des revendications 12 à 14, **caractérisée en ce que** ledit récepteur à sept domaines transmembranaires est un récepteur olfactif.

## Patentansprüche

1. Expressionskassette, umfassend:
a) einen Promoter, der vom Promoter des Polyhedrins eines Baculovirus durch Deletion der ganzen Region oder eines Teils der Region des Promoters, die sich von Position-1 bis Position -12 bezüglich der Initiationsstelle der Translation des Polyhedrins erstreckt, abgeleitet ist;
b) eine Sequenz, die für einen Rezeptor mit sieben Transmembrandomänen codiert, die unter der Transkriptionskontrolle des genannten Promoters steht.

2. Expressionskassette nach Anspruch 1, **dadurch gekennzeichnet, dass** sie außerdem stromaufwärts zu Sequenz b) eine Sequenz, die für ein Peptidsignal codiert, umfasst.

3. Expressionskassette nach einem der Ansprüche 1 oder 2, **dadurch gekennzeichnet, dass** der genannte Rezeptor mit sieben Transmembrandomänen ein olfaktiver Rezeptor ist.

4. Verfahren zum Expremieren eines Rezeptors mit sieben Transmembrandomänen in einer Insektenzelle, **dadurch gekennzeichnet, dass** man die Insektenzelle mit einem rekombinanten Baculovirus infiziert, das eine Expressionskassette nach einem der Ansprüche 1 bis 3 umfasst.

5. Verfahren nach Anspruch 4, **dadurch gekennzeichnet, dass** man in derselben Insektenzelle auch ein Protein G exprimiert.

6. Verfahren nach Anspruch 5, **dadurch gekennzeichnet, dass** das Protein G unter der Kontrolle des Promoters des Gens P10 eines Baculovirus exprimiert wird.

7. Verfahren nach Anspruch 6, **dadurch gekennzeichnet, dass** man ein doppelt rekombinantes Baculovirus verwendet, umfassend:
- eine Expressionskassette nach einem der Ansprüche 1 bis 3 und
- eine Sequenz, die für ein Protein G kodiert, die unter der Transkriptionskontrolle des Promoters des Gens P10 des genannten Baculovirus steht.

8. Rekombinantes Baculovirus, das eine Expressionskassette nach einem der Ansprüche 1 bis 3 umfasst.

9. Rekombinantes Baculovirus nach Anspruch 8, **dadurch gekennzeichnet, dass** die Expressionskassette als Ersatz für den Promoter und das Gen des Polyhedrin des Baculovirus insertiert ist.

10. Rekombinantes Baculovirus nach Anspruch 9, **dadurch gekennzeichnet, dass** das Baculovirus außerdem eine Sequenz umfasst, die für ein Protein G kodiert, die unter die Transkriptionskontrolle des Promoters des Gens P10 gestellt ist.

11. Insektenzelle, die durch ein rekombinantes Baculovirus nach einem der Ansprüche 8 bis 10 infiziert ist.

12. Verwendung einer Insektenzelle nach Anspruch 11 zur Bestimmung der Funktionalität eines vermeintlichen Rezeptors mit sieben Transmembrandomänen.

13. Verwendung einer Insektenzelle nach Anspruch 11 zur Identifizierung des Liganden (der Liganden) eines orphanen (verwaisten) Rezeptors mit sieben Transmembrandomänen.

14. Verwendung einer Insektenzelle nach Anspruch 11 zur Identifizierung eines Rezeptors (von Rezeptoren) mit sieben Transmembrandomänen, die fähig sind, an einen Liganden von Interesse zu binden.

15. Verwendung nach einem der Ansprüche 12 bis 14, **dadurch gekennzeichnet, dass** der Rezeptor mit sieben Transmembrandomänen ein olfaktiver Rezeptor ist.

## Claims

1. Expression cassette comprising:
a) a promoter derived from the polyedrin promoter of a baculovirus by the deletion of all or part of the region of said promoter that extends from position -1 to position -12 relative to the polyedrin translation initiation site; and
b) a sequence coding for a seven-transmembrane-domain receptor, placed under the transcriptional control of said promoter.

2. Expression cassette according to Claim 1, **characterized in that** it also comprises, upstream from the sequence b), a sequence coding for a signal peptide.

3. Expression cassette according to Claim 1 or 2, **characterized in that** said seven-transmembrane-domain receptor is an olfactory receptor.

4. Method of expressing a seven-transmembrane-domain receptor in an insect cell, **characterized in that** said insect cell is infected with a recombinant baculovirus comprising an expression cassette according to any one of Claims 1 to 3.

5. Method according to Claim 4, **characterized in that** a G protein is also expressed in the same insect cell.

6. Method according to Claim 5, **characterized in that** said G protein is expressed under the control of the promoter of the P10 gene of a baculovirus.

7. Method according to Claim 6, **characterized in that** a double-recombinant baculovirus is used which comprises:
- an expression cassette according to any one of Claims 1 to 3; and
- a sequence coding for a G protein, placed under the transcriptional control of the promoter of the P10 gene of said baculovirus.

8. Recombinant baculovirus comprising an expression cassette according to any one of Claims 1 to 3.

9. Recombinant baculovirus according to Claim 8, **characterized in that** said expression cassette is inserted in place of the polyedrin promoter and gene of said baculovirus.

10. Recombinant baculovirus according to Claim 9, **characterized in that** said baculovirus also comprises a sequence coding for a G protein, placed under the transcriptional control of the promoter of the P10 gene.

11. Insect cell infected with a recombinant baculovirus according to any one of Claims 8 to 10.

12. Use of an insect cell according to Claim 11 for determining the functionality of a putative seven-transmembrane-domain receptor.

13. Use of an insect cell according to Claim 11 for identifying the ligand(s) of an orphan seven-transmembrane-domain receptor.

14. Use of an insect cell according to Claim 11 for identifying one or more seven-transmembrane-domain receptors capable of binding to a ligand of interest.

15. Use according to any one of Claims 12 to 14, **characterized in that** said seven-transmembrane-domain receptor is an olfactory receptor.
